# EUROPEAN PATENT SPECIFICATION

(11) **EP 2 007 446 B1**
(45) Date of publication and mention of the grant of the patent: **12.09.2012**
(21) Application number: 07874488.5
(22) Date of filing: 20.03.2007
(51) Int. Cl.: A61L 27/28, A61L 27/50, A61L 29/08, A61L 29/14, A61L 31/08, A61L 31/14, A61F 2/02, B05C 3/09

(54) **METHODS AND DEVICES HAVING ELECTRICALLY ACTUATABLE SURFACES**
VERFAHREN UND VORRICHTUNGEN MIT ELEKTRISCH ANTREIBBAREN OBERFLÄCHEN
PROCÉDÉS ET DISPOSITIFS DOTÉS DE SURFACES ACTIONNABLES ÉLECTRIQUEMENT

(30) Priority: 24.03.2006 US 388301
(43) Date of publication of application: 31.12.2008
(73) Proprietor: Boston Scientific Limited, Christ Church (BB)
(72) Inventor: SAHATJIAN, Ronald, A., Lexington, MA 02420 (US); O'SHEA, Timothy, J., Hopkinton, MA 01748 (US)
(74) Representative: Hauck Patent- und Rechtsanwälte
(86) International application number: PCT/US2007/006935
(87) International publication number: WO 2008/127224

(56) References cited:
- WO-A-2006/028764
- LAHANN K, MITRAGOTRI S, TRAN TN, KAIDO H, SUNDARAM J, CHOI I, HOFFER S, SOMORJAI G, LANGER R: "A reversible Switching Surface" SCIENCE, vol. 299, 2003, pages 371-374, XP002490005 cited in the application

## Description

### FIELD OF THE INVENTION

The present invention relates to the field of insertable or implantable medical devices, such as balloon catheters, stents and other similar diagnostic or therapeutic devices, which may be provided within the body for treatment and diagnosis of diseases and conditions. In particular, the present invention relates to devices whose surfaces are electrically actuatable between a hydrophobic state and a less hydrophobic state or a hydrophilic state.

### BACKGROUND OF THE INVENTION

Numerous medical devices have been developed for the delivery of therapeutic agents to the body. The desired release profile for the therapeutic agent is dependent upon the particular treatment at hand, including the specific condition being treated or prevented, the specific site of administration, the specific therapeutic agent selected, and so forth.

In accordance with some typical delivery strategies, a therapeutic agent is provided within a polymeric carrier layer and/or beneath a polymeric barrier layer that is associated with a medical device. Once the medical device is placed at the desired location within a patient, the therapeutic agent is released from the medical device at a rate that is dependent upon the nature of the polymeric carrier and/or barrier layer.

Implantation of vascular stents is a prime example of a situation where local drug therapy is needed, but where it is possible that the drugs will produce unwanted systemic side effects. For example, endovascular stents are placed in the dilated segment of a vessel lumen to mechanically block the effects of abrupt closure and restenosis. Recent developments have led to stents which attempt to provide anti-restenotic agents and/or other medications such as anti-thrombosis agents to regions of a blood vessel which have been treated by angioplasty or other interventional techniques.

However, an ongoing issue with the drug release coatings that are presently applied to devices such as stents is achieving a therapeutic concentration of a drug locally at a target site within the body without potentially producing unwanted systemic side effects. For instance, because the stent is placed within a flowing blood stream during placement and upon implantation, potential unwanted systemic effects may result from the premature release of undesirable quantities of the drug into the blood stream. Further, if quantities of therapeutic substance are released into the blood stream during positioning of the stent, less substance is available for actual local treatment when the stent is expanded, resulting in the potential for inadequate local dosing.

In the prior art are taught various attempts at devices which control the elution of a drug from a drug-loaded device such as a stent. For example, U.S. Patent No. 6,419,692 by Yang et al discloses polymeric layered catheters, wherein a protective outer polymer coating prevents elution of a drug-containing layer until expansion of the catheter causes fissures in the outer coating that allows exposure of the drug- containing layer. Also, U.S. Patent No. 5,972,027 by Johnson discloses a porous metal stent wherein pores within the stent are loaded with a drug that is released in the body.

Although controlled release of a therapeutic agent has existed in various forms for several years, there is nonetheless a continuing need for improved and more precise drug delivery systems that address the need for greater control of the release of the therapeutic substance during implantation and following implantation. There especially exists a need to provide precise drug delivery systems whose release characteristics may be readily modulated in situ, depending on the required needs and conditions.

There is also a continuing need for medical devices which have controllable lubricity such that the devices are able to be moved smoothly within the body cavity or vessel during introduction into and removal from the body, while at the same time being able to be positioned precisely without shifting or movement when placed at a target site. Although various lubricious coatings have been taught for many years, there is still a long-standing need for methods and devices wherein the lubricity can be adjusted or modulated depending on changing in situ needs and conditions.

From WO 2006/028764 A1 a method for coating at least a portion of a medical device has become known. The medical device has an insulator layer overlying an electrode on the insulator layer. The method includes arranging a liquid agent on at least the portion of the medical device. The method further includes applying an electrostatic potential to the electrode and applying another electrostatic potential to the liquid agent. A medical appliance having a coating applied by a method is provided. A system is provided for applying a liquid coating to a medical device.

### SUMMARY OF THE INVENTION

These and other challenges of the prior art are addressed by the present invention.

According to an aspect of the invention, an implantable or insertable medical device is provided, which comprises a surface region that is electrically actuatable between a hydrophobic state and a less hydrophobic state or a hydrophilic state. Such devices are advantageous, for example, in that the surface lubricity of the device may be modulated in vivo or ex vivo. Such devices are also advantageous, for example, in that drug delivery may be modulated by varying the hydrophobicity/ hydrophilicity of the surface region in vivo.

These and other embodiments and advantages of the present invention will become immediately apparent to those of ordinary skill in the art upon review of the Detailed Description and Claims to follow.

### BRIEF DESCRIPTION OF THE DRAWINGS

FIG. 1 is a schematic representation of a catheter according to one embodiment of the present invention having a folded balloon as it is being inserted within a body lumen.
FIG. 2 is a schematic representation in a magnified see-through view of the catheter of FIG. 1 having the balloon in an expanded position at a target vessel within the body.
FIG. 3 is one embodiment of the catheter of FIG. 2 comprising a drug-eluting balloon catheter showing a close-up of a section A of the wall of the catheter of FIG 2 that has been positioned within a target vessel in the body. The drug-containing catheter is covered with finger-like projections having an overlying hydrophobic layer.
FIG. 4A-4B is a close-up of a longitudinal section A of the catheter of FIG. 2 according to two embodiments. Electrical energy is applied to the catheter generating an electric field through the wall of the balloon catheter causing contact between the therapeutic agent of the catheter and the surrounding body fluid.
FIGS. 5A-5C are schematic representations of balloon catheters according to three embodiments of the present invention showing different configurations for placement of the coating containing finger-like projections having an overlying hydrophobic layer.
FIG. 6 is a perspective view of a drug-eluting stent in accordance with an exemplary embodiment of the present invention.
FIG. 7A is a magnified, partial perspective view of FIG. 6.
FIG. 7B is a magnified, cross-sectional view of FIG. 7A across line B-B illustrating the coating applied to wire members of the stent.
FIGS. 8A and 8B are schematic illustrations of a layer of molecules on a positively charged and negatively charged substrate, respectively. The molecules have a hydrophobic chain portion (portion with ball tip) and a charged polar portion.
Fig. 9A is a schematic, longitudinal, cross-sectional view of the distal end of a balloon catheter as it is advanced over a guidewire, in accordance with an embodiment of the present invention. Figs. 9B and 9C are schematic, axial, cross-sectional views of the balloon catheter of Fig. 9A, taken along planes B-B and C-C, respectively.

### DETAILED DESCRIPTION OF THE EMBODIMENTS

A more complete understanding of the method and apparatus of the present invention is available by reference to the following detailed description of the embodiments when taken in conjunction with the accompanying drawings. The detailed description of the embodiments which follows is intended to illustrate but not limit the invention.

The present invention relates to implantable or insertable medical devices, which contain one or more surface regions that are electrically actuatable between a hydrophobic state and a less hydrophobic state or a hydrophilic state.

Hydrophobic surfaces are defined herein as surfaces having a static water contact angle that is greater than 90°, for example ranging from 90° to 100° to 110° to 120° degrees or more. Hydrophilic surfaces are defined herein as surfaces having a static water contact angle that is less than or equal to 90°, for example, ranging from 90° to 75° to 50° to 25° to 10° to 5° or less.

In some embodiments of the invention, the surfaces are electrically actuatable between a superhydrophobic state and a hydrophilic state. For purposes of the present invention, a superhydrophobic surface is one that displays static water contact angles above 140° (e.g., ranging from 140° to 150° to 155° to 160° to 165° to 170° to 175° to 180°).

Various instruments are available for measuring static contact angles, for example, the PG-3 PocketGoniometer contact angle tester from Thwing-Albert Instrument Company Philadelphia, PA, USA or the Phoenix 450 Contact Angle Analyzer available from AhTECH LTS Co., Ltd., Korea.

A typical way of creating hydrophobicity is to employ materials with low surface energy, such as fluorocarbon polymers. Low energy materials are defined herein as those that display static water contact angles that are greater than 90°. However, even fluorocarbon materials yield water contact angles that are only around 120° or so, far short of a superhydrophobic surface as defined herein. Nevertheless, surfaces with substantially greater water contact angles do exist in nature, and they have been created in the laboratory. In general, in addition to being formed using low surface energy (inherently hydrophobic) materials, these surfaces have been shown to have microscale and/or nanoscale surface texturing. One superhydrophobic biological material commonly referred to in the literature is the lotus leaf, which has been observed to be textured with 3-10 micron hills and valleys, upon which are found nanometer sized regions of hydrophobic material.

Exemplary hydrophobic polymers which are suitable for use in the present invention can be selected from, for example, but not limited, by the following hydrophobic monomers: vinyl aromatic monomers, including unsubstituted vinyl aromatics, vinyl substituted aromatics, and ring-substituted vinyl aromatics; vinyl esters, vinyl halides, alkyl vinyl ethers, and other vinyl compounds such as vinyl ferrocene; aromatic monomers other than vinyl aromatics, including acenaphthalene and indene; acrylic monomers, including alkyl acrylates, arylalkyl acrylates, alkoxyalkyl acrylates, halo-alkyl acrylates, and cyano-alkyl acrylates; methacrylic monomers, including methacrylic acid esters (methacrylates) and other methacrylic-acid derivatives including methacrylonitrile; acrylic monomers, including acrylic acid esters and other acrylic-acid derivatives including acrylonitrile, alkyl methacrylates and aminoalkyl methacrylates; alkene-based monomers, including ethylene, isotactic propylene, 4-methyl pentene, 1-octadecene, and tetrafluoroethylene and other unsaturated hydrocarbon monomers; cyclic ether monomers; ether monomers other than acrylates and methacrylates; and other monomers including epsilon-caprolactone; and (2) hydrophilic monomers including the following: vinyl amines, alkyl vinyl ethers, 1-vinyl-2-pyrrolidone and other vinyl compounds; methacrylic monomers including methacrylic acid and methacrylic acid salts; acrylic monomers such as acrylic acid, its anhydride and salt forms, and acrylic acid amides; alkyl vinyl ether monomers such as methyl vinyl ether; and cyclic ether monomers such as ethylene oxide.

Surface regions that are electrically actuatable between a hydrophobic state and a hydrophilic state can be constructed in various ways.

For example, in certain embodiments of the invention, the surface region may comprise a layer of amphiphilic molecules that comprise (i) a hydrophobic chain portion that is covalently or non-covalently attached to the surface of a conductive region and (ii) a charged polar portion. The charged polar portion is (a) drawn to the conductive region when the conductive region has a charge whose sign is opposite to that of the charged polar portion and (b) is repelled from the conductive region when the conductive region has a charge whose sign the same as that of the charged polar portion. Typically, the charged polar portion is attached to one end of the hydrophobic chain portion, whereas the other end of the end of the hydrophobic chain portion is attached to the conductive region. As defined here, conductive materials include both conductive and semi-conductive materials. Examples of conductive materials include metals, metal alloys, semiconductors, conductive polymers and so forth.

Charge may be introduced, for example, by application of a suitable voltage between the conductive region and an electrode configured for electrical contact with the body, for example, being provided on a surface of said medical device or being implantable or insertable in a manner independent of the medical device. The electrode may be formed form any suitable conductive material and may be selected, for example, from those listed elsewhere herein.

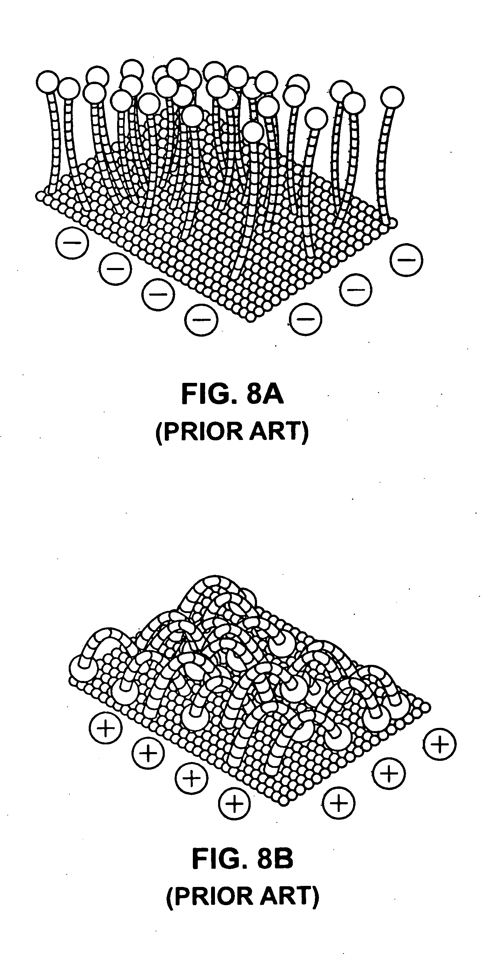

One example of a surface of this type is found, for example, in J. Lahann et al., "A Reversibly Switching Surface," Science, vol. 299, 17 January 2003, 371-374, which describes a process whereby a monolayer of a molecule containing a chain-like hydrophobic portion and a charged end group, specifically an anionic group, is assembled on a conductive substrate. As shown schematically in Fig. 8A, when the conductive substrate 800 is supplied with a negative charge, the anionic groups 820 are repelled from the substrate, presenting a hydrophilic surface to the surrounding environment. When the conductive substrate 800 is supplied with a positive charge as shown in Fig. 8B, however, the anionic groups 820 are drawn toward the substrate surface, causing the molecules to bend over and present the chain-like hydrophobic portions 810 to the surrounding environment. As a result, the surface has controlled wettability. More specifically, (16-Mercapto) hexadecanoic acid (MHA) was chosen because it (i) self-assembles on Au(1 1 1) into a monolayer and (ii) has a hydrophobic chain capped by a negatively charged, hydrophilic carboxylate group. To create a monolayer with sufficient spacing between the individual MHA molecules to allow the molecules to bend over, they resorted to and self-assembly of a MHA derivative with a globular end group. Subsequent cleavage of the end groups resulted in a monolayer of the MHA, which can then be switched between hydrophilic and hydrophobic states as discussed above.

Another type of electrowetting is commonly observed when a droplet of conductive liquid is placed onto a dielectric coated conductor surface, and a voltage is applied across the dielectric coating such that the droplet flattens and spreads on the surface. While not wishing to be bound by theory, it is believed that electrowetting is a phenomenon that relates changes in surface interfacial energy in the presence of an electric field. More particularly, as the electric field is applied across the dielectric material, the conductive liquid droplet above the dielectric surface experiences a change in surface interfacial energy and thus a change in the droplet's equilibrium contact angle. Typically, the presence of the electric field results in a reduction of surface interfacial energy and hence the contact angle. The dynamics of the wetting behavior of liquids on nanostructured surfaces is discussed in T.N. Krupenkin et al., "From Rolling Ball to Complete Wetting: The Dynamic Tuning of Liquids on Nanostructured Surfaces," Langmuir, Vol. 20, No. 10, 2004, p. 3824.

In certain embodiments of the invention, the surface regions of the medical devices comprise a conductive region coated with a dielectric layer. (As used herein a "layer" of a given material is a region of that material whose thickness is small compared to both its length and width. As used herein a layer need not be planar, for example, taking on the contours of an underlying substrate. Layers can be discontinuous, e.g., patterned. Terms such as "film," "layer" and "coating" may be used interchangeably herein.) Preferably, such devices are provided with a low-surface-energy material, which is inherently hydrophobic. Such a surface may be provided, for example, by selecting a hydrophobic dielectric coating material. (Alternatively, a surface of this type may be provided, for example, by providing a hydrophobic coating over the dielectric coating.) Examples of dielectrics include metal and semiconductor oxides and nitrides, polymers, and so forth. Examples of hydrophobic materials include fluorine containing polymers (fluoropolymers), and polysiloxanes (e.g., silicones) among others.

Subsequent application of a suitable voltage across the hydrophobic dielectric coating (or across the hydrophobic/dielectric coating combination), for example by applying a voltage between the conductive region of the device and an electrode on the opposite side of the dielectric coating (or the hydrophobic/dielectric coating combination) from conductive region, may then be employed to cause the surface to switch from a hydrophobic state to a less hydrophobic state or even a hydrophilic state.

The electrode is therefore configured for electrical contact with the body, for example, being provided on a surface of said medical device or being implantable or insertable in a manner independent of the medical device. The electrode may be formed form any suitable conductive material, such as those listed elsewhere herein.

In certain embodiments of the invention, the surface regions may comprise a plurality of micro- or nano-protrusions (e.g., columns, pillars, finger-like projections, etc.), which further comprise a hydrophobic dielectric coating material (or a dielectric coating layer which is provided with a hydrophobic coating). As defined herein, a nano-protrusion is a protrusion whose largest lateral dimension (e.g., the diameter for a column, the width for a standing plate-like structure, etc.) ranges between 0.1 and 100 nm in length, whereas a micro-protrusion is a protrusion whose largest lateral dimension ranges between 0.1 and 100 µm. Such protrusions may be high aspect ratio structures whose height is at least as great as its largest lateral dimension, including 1 to 2 to 5 to 10 to 20 to 50 or more times as great. Protrusions can be regular or irregular in cross-section, having for example, a circular cross-section (e.g., a cylindrical column, cone, etc.), a square cross-section (e.g., a square column, pyramid, etc.), as well as a wide variety of other cross-sections, including oval cross-sections, triangular cross-sections, rectangular cross-sections, trapezoidal cross-sections, pentagonal cross-sections, and so forth.

Surfaces of this nature have been reported to be electrically actuatable between a superhydrophobic state and a less hydrophobic or hydrophilic state based on electrowetting. For example, Krupenkin et al. describe nanostructured superhydrophobic surfaces, which were constructed by etching a microscopic array of cylindrical nanoposts into the surface of a silicon wafer. Each post had a diameter of about 350 nm and a height of about 7 µm (or an aspect ratio of about 20). The distance between posts (pitch) varied from I to 4 µm. An oxide layer was thermally grown to provide electrical isolation between the substrate and the liquid. A thin conformal layer of a low-surface-energy polymer was then deposited to create the hydrophobic surface. In the absence of any applied potential, high surface tension liquids such as water and molten salt, each resulted in a highly mobile ball having high contact angles. Upon the application of a sufficient potential between the liquid and the silicon substrate, however, the ball experienced a sharp transition to an immobile droplet state. For the rolling ball state, there was little to no penetration of liquid in the nanostructured layer. For the immobile droplet, the liquid penetrated all the way to the bottom of the nanostructured layer, dramatically increasing the liquid-solid interfacial area.

The ability to transition medical device surface regions between a hydrophobic state (including a superhydrophobic state) and a less hydrophobic state or a hydrophilic state is advantageously utilized in conjunction with drug delivery.

For example, switching medical device surface regions from hydrophobic to hydrophilic has been shown to result in more intimate interaction between the surface and water. *See, e.g.,* Krupenkin et al., *supra.* This effect is utilized in certain embodiments of the invention to bring a fluid (e.g., a bodily fluid such as urine, blood, etc.) into more intimate contact with a therapeutic-agent-containing reservoir, thereby permitting or increasing the mass transport of the therapeutic agents (also referred to herein as a "drugs").

Therapeutic agents may be selected, for example, from the following: adrenergic agents, adrenocortical steroids, adrenocortical suppressants, alcohol deterrents, aldosterone antagonists, amino acids and proteins, ammonia detoxicants, anabolic agents, analeptic agents, analgesic agents, androgenic agents, anesthetic agents, anorectic compounds, anorexic agents, antagonists, anterior pituitary activators and suppressants, anthelmintic agents, anti-adrenergic agents, anti-allergic agents, anti-amebic agents, anti-androgen agents, anti-anemic agents, anti-anginal agents, anti-anxiety agents, anti-arthritic agents, anti-asthmatic agents, anti-atherosclerotic agents, antibacterial agents, anticholelithic agents, anticholelithogenic agents, anticholinergic agents, anticoagulants, anticoccidal agents, anticonvulsants, antidepressants, antidiabetic agents, antidiuretics, antidotes, antidyskinetics agents, anti-emetic agents, anti-epileptic agents, anti-estrogen agents, antifibrinolytic agents, antifungal agents, antiglaucoma agents, antihemophilic agents, antihemophilic Factor, antihemorrhagic agents, antihistaminic agents, antihyperlipidemic agents, antihyperlipoproteinemic agents, antihypertensives, antihypotensives, anti-infective agents, anti-inflammatory agents, antikeratinizing agents, antimicrobial agents, antimigraine agents, antimitotic agents, antimycotic agents, antineoplastic agents, anti-cancer supplementary potentiating agents, antineutropenic agents, antiobsessional agents, antiparasitic agents, antiparkinsonian drugs, antipneumocystic agents, antiproliferative agents, antiprostatic hypertrophy drugs, antiprotozoal agents, antipruritics, antipsoriatic agents, antipsychotics, antirheumatic agents, antischistosomal agents, antiseborrheic agents, antispasmodic agents, antithrombotic agents, antitussive agents, anti-ulcerative agents, anti-urolithic agents, antiviral agents, benign prostatic hyperplasia therapy agents, blood glucose regulators, bone resorption inhibitors, bronchodilators, carbonic anhydrase inhibitors, cardiac depressants, cardioprotectants, cardiotonic agents, cardiovascular agents, choleretic agents, cholinergic agents, cholinergic agonists, cholinesterase deactivators, coccidiostat agents, cognition adjuvants and cognition enhancers, depressants, diagnostic aids, diuretics, dopaminergic agents, ectoparasiticides, emetic agents, enzyme inhibitors, estrogens, fibrinolytic agents, free oxygen radical scavengers, gastrointestinal motility agents, glucocorticoids, gonad-stimuiating principles, hemostatic agents, histamine H2 receptor antagonists, hormones, hypocholesterolemic agents, hypoglycemic agents, hypolipidemic agents, hypotensive agents, HMGCoA reductase inhibitors, immunizing agents, immunomodulators, immunoregulators, immunostimulants, immunosuppressants, impotence therapy adjuncts, keratolytic agents, LHRH agonists, luteolysin agents, mucolytics, mucosal protective agents, mydriatic agents, nasal decongestants, neuroleptic agents, neuromuscular blocking agents, neuroprotective agents, NMDA antagonists, non-hormonal sterol derivatives, oxytocic agents, plasminogen activators, platelet activating factor antagonists, platelet aggregation inhibitors, post-stroke and post-head trauma treatments, progestins, prostaglandins, prostate growth inhibitors, prothyrotropin agents, psychotropic agents, radioactive agents, repartitioning agents, scabicides, sclerosing agents, sedatives, sedative-hypnotic agents, selective adenosine A1 antagonists, serotonin antagonists, serotonin inhibitors, serotonin receptor antagonists, steroids, stimulants, thyroid hormones, thyroid inhibitors, thyromimetic agents, tranquilizers, unstable angina agents, uricosuric agents, vasoconstrictors, vasodilators, vulnerary agents, wound healing agents, xanthine oxidase inhibitors, and the like.

Specific examples of therapeutic agents include paclitaxel, sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten-NG, Ap- 17, abciximab, clopidogrel, Ridogrel, beta-blockers, bARKct inhibitors, phospholamban inhibitors, and Serca 2 gene/protein among others, many of which are anti-restenotic agents. Numerous additional therapeutic agents useful for the practice of the present invention are also disclosed in U.S. Patent Application 2004/0175406.

A wide range of therapeutic agent loadings can be used in connection with the medical devices of the present invention, with the therapeutically effective amount being readily determined by those of ordinary skill in the art and ultimately depending, for example, upon the condition to be treated, the age, sex and condition of the patient, the nature of the therapeutic agent, the nature of the composite region(s), the nature of the medical device, and so forth.

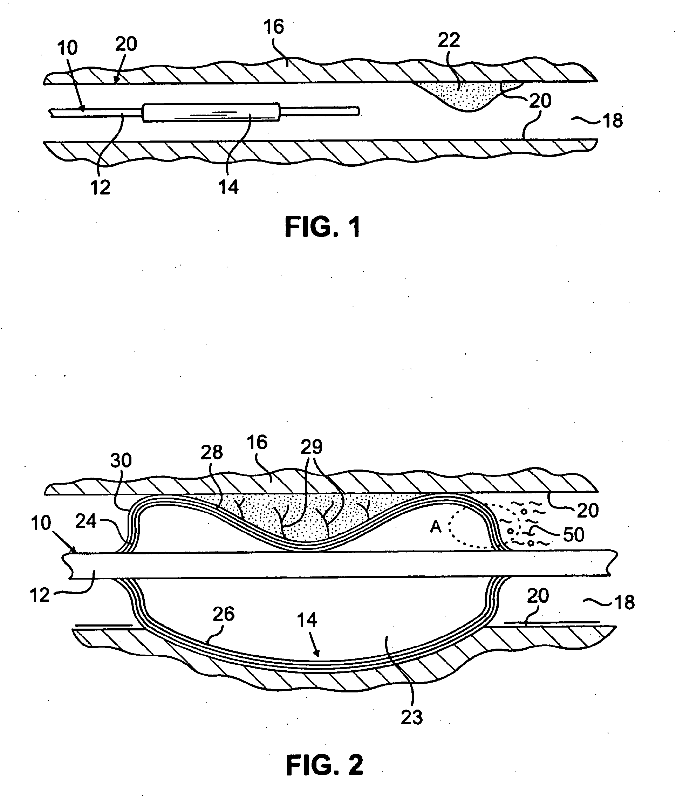

Now referring to the drawings, wherein like reference numerals refer to like elements throughout several views, FIGS. 1 and 2 depict the incorporation of surface regions in accordance with the present invention into a balloon catheter design. Referring to FIG. 1, an illustrated embodiment of a balloon catheter 10 of the present invention is shown to include an elongated tubular member 12 having disposed on a distal portion thereof an inflatable balloon 14. The balloon 14 is shown within a vessel 16 within the vascular system with a lumen 18 that is defined by lumen walls 20. As shown, the lumen 18 is partially blocked by a stenosis or thrombosis 22. The balloon catheter 10 of FIG. 1 is depicted in a folded or uninflated profile which is suitable for inserting into the lumen 18 of the blood vessel 16. Referring now to FIG. 2, the balloon catheter 10 of FIG. 1 is shown in an expanded state located across the stenotic area 22.

As shown in FIG. 2, the balloon 14 having an inner lumen 23 includes a conductive layer 24, such as a metallic layer, metallic wire or circuit traces, attached to the wall 26 of the balloon 14 which is capable of transmitting energy, e.g. electrical energy, to the balloon 14. In certain embodiments, the conductive layer 24 comprises a plurality of elements attached to the wall 26 of the balloon 14 which are capable of transmitting electrical energy. As also shown in FIG. 2, the balloon in certain embodiments also includes a reservoir such as a drug-containing layer 28 which includes a drug or therapeutic substance 29. Overlying the drug-containing layer 28 is a surface-modulating region 30 (e.g., an electrically actuatable surface region).

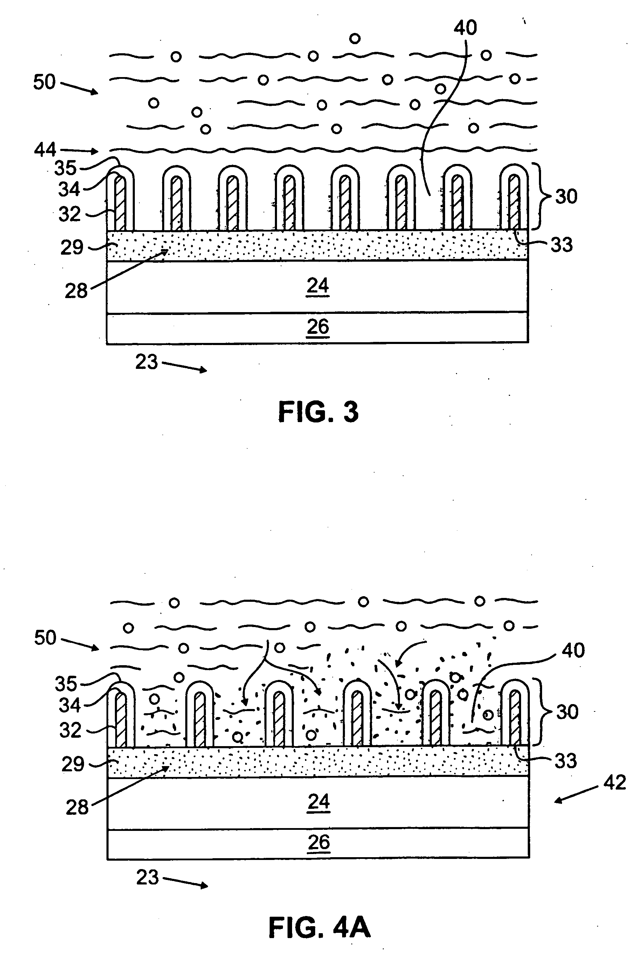

FIG. 3 is a close-up of a longitudinal section A of the wall of the catheter of FIG. 2 that has been positioned within a target vessel in the body wherein the surface-modulating region 30 is adjacent body fluid or blood 50 contained within the vessel, and a boundary layer 44 is observed between the body fluid or blood 50 and the surface-modulating region 30. As shown in FIG. 3, in one embodiment, the surface-modulating region 30 comprises two components: a plurality of electrically conductive protrusions such as columns 32, each having a base 33 and a tip 34 and hydrophobic dielectric coating such as a hydrophobic polymeric coating 35 (e.g., a fluorinated or silicone polymer) overlying at least a portion of the surface of conductive columns 32. Such columns 32 may be formed from a variety of conductive materials including metals, metal alloys and semiconductors such as silicon. As shown in FIG. 3, the columns 32 are in electrical communication with the conductive layer 24 through a drug-containing layer 28 containing drug 29, and the conducting layer 24 in also directly adjacent to the balloon wall 26.

In certain embodiments, a layer of dielectric material (not shown) may be further provided between the conductive columns 32 and a hydrophobic polymer coating 35 as noted above. As a specific example, the protrusions may comprise conductive columns that comprise silicon, the dielectric material layer may comprise a silicon compound, e.g., SiO₂, and the hydrophobic polymeric may comprises a fluorinated polymer such as, but not including but not limited to polytetrafluoroethylenes (PTFE) or a silicone such as poly(dimethylsiloxane) (PDMS). Like fluoropolymers, PDMS is intrinsically extremely hydrophobic.

In some embodiments, the electrically conductive columns 32 may comprise a plurality of columns or other finger-like projections that are aligned to form an array. In one exemplary embodiment, the conductive columns 32 are patterned and coated with a thin hydrophobic dielectric layer. The conductive columns 32 have a typical height ranging form about 1 to about 10 µm, more typically about 7 µm and a column diameter (for a circular column) of about 100 to 500 nm, more typically about 350 nm. The distance between columns 32 (pitch) may vary, for example, from about 1 to 5 µm, among other ranges. As shown in FIG. 4A, by supplying electrical energy 42, e.g., voltage of suitable magnitude and polarity between the body fluid 50 and the columns 32, the surface temporarily behaves as if it were a hydrophilic surface, drawing the body fluid 50 into the channel regions 40 between the columns 32, where it comes into contact with the drug-containing layer 28, promoting transport of the drug 29 therefrom. A typical voltage suitable for causing the desired electrowetting effects is about 10-25 V.

In the field of microelectronics and microfluidics, researchers have been utilizing electric fields to control dynamically the wetting properties of a surface to transport fluids in micrometer-sized channels. Certain embodiments of the invention also use the principle of electrowetting using electric fields for driving or pumping liquid segments on a surface or through a channel. With reference to Fig. 4A, as electrical energy 42 is applied to the conductive layer 24, an electrical field is generated between the conducting column 32 and the blood or body fluid. Aqueous liquid such as blood or body fluid 50 normally will not penetrate or "wet" the channels 40 defined by the hydrophobic polymer coated conducting columns 32, wherein "channels" refers to the spaces between the columns. In contrast, without application of such electrical energy 42, as shown in FIG. 3, a boundary layer 44 is observed across the surface-modulating region 30. Upon applying an electric potential between the blood 50 and the conducting column 32, the surface-modulating layer region 30 becomes hydrophilic, causing the blood to advance through the channels 40 and wet the entire region of the conducting columns 32 and the drug-containing layer 28 above the electrically activated conducting layer 24.

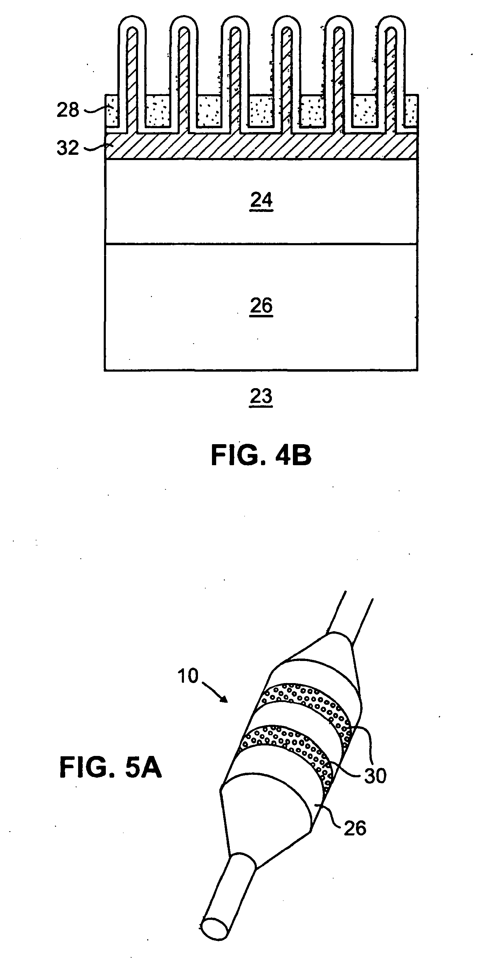

In certain other embodiments, as shown in FIG. 4B, the conductive layer 24 and the conductive columns 32 are in electrical communication with one another. The conductive columns 32 extend through the drug-containing layer 28 such that the base 33 of the columns is directly adjacent the conductive layer 24. As indicated previously, release of a therapeutic substance may be enhanced by contact with water or other liquids present in the blood or other body lumen. When the surface-modulating region 30 is "wetted," the blood or body fluid 50 comes into contact with the drug-containing layer 28, which may cause the drug 29 to be released by diffusion, dispersion, disintegration or dissolution. The therapeutic agent or drug 29 may be, for example, solid or liquid, in the form of, for example, powders such as nanoparticles or dry or wet coatings (e.g., gels) and may be in a discrete form or integrated into the structure of the drug-containing layer 28 in a carrier matrix.

In certain aspects and embodiments of the invention, the selected therapeutic agents are charged therapeutic agents. By "charged therapeutic agent" is meant a therapeutic agent that has an associated charge. For example, a therapeutic agent may have an associated charge (a) because it is inherently charged (e.g., because it is an acid, base, or salt), (b) because it has been modified to carry a charge by covalently linking a charged species to it, (c) because it is non-covalently linked to a charged species (e.g., based on hydrogen bonding with the charged species, or because it forms complexes and/or coordinative bonds with charged species), or (d) because it is attached to or encapsulated within a charged particle, such as a charged nanoparticle (i.e., a charged particle of 100 nm or less in diameter), including nanocapsules and charged micelles, among others. Taking paclitaxel as one specific example, various cationic and anionic forms of paclitaxel are known. See for example, U.S. Patent No. 6,730,699.

In certain embodiments, the entire surface of the balloon wall 26 is coated with a drug-containing layer 28 (which may, for example, consist of the drug in pure form or admixed with another substance, and which may, for example, be in the form of a collection of particles or in the form of drug-containing layer). In other embodiments, only certain portions of the wall 26 of the balloon 14 are coated with a drug-containing layer 28.

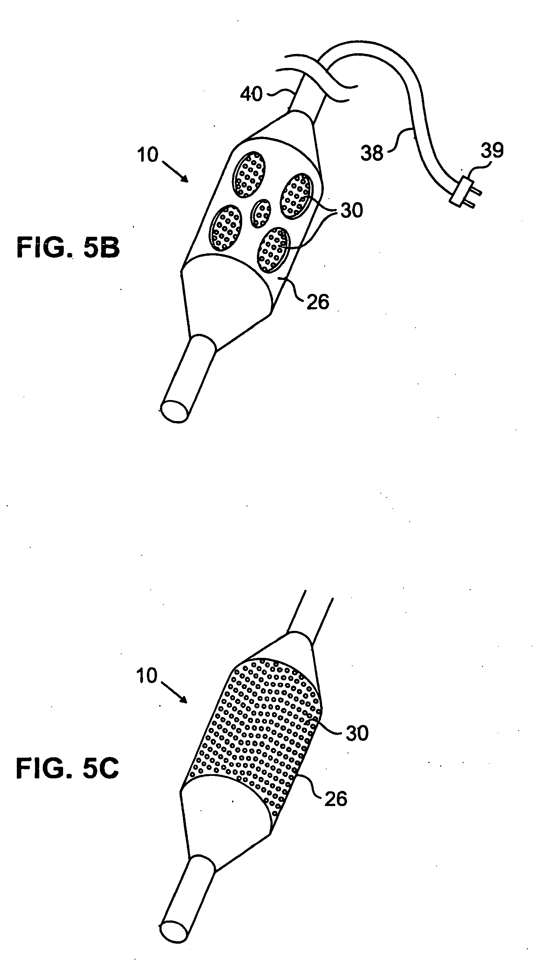

FIGS. 5A, 5B, and 5C are perspective views of balloon catheters 10 wherein discrete portions of the surface 26 of the balloon 14 are covered with surface- modulating regions 30, which may be associated with drug-containing layers 28 as described above.

As would be appreciated by one of skill in the art, a wire (not shown) for providing power to the conductive layer(s) 24 on the balloon 14 may, for example, extend as a trace along the wall 26 of the balloon 14 or may be encapsulated or otherwise disposed within the body of the catheter 10. The wire connects to an electrical cable 38 that extends from the proximal end 40 of the balloon 14. FIG. 5B shows that the cable 38 ends with a plug 39 that connects with an energy source and appropriate conventional catheter control equipment (not shown). Alternatively, the devices of the present invention may be battery-powered or remotely powered using standard wireless or passive sensor technology known to one of skill in the art. For example, several types of wireless or passive sensors that harvest RF energy have been developed for various in vivo applications such as measuring intraocular, intracranial and arterial pressure. See LaVan et al., Small-scale systems for in vivo drug delivery, Nature Biotechnology 21 (10): 1 184-1 191 (October 2003).

With respect to the conductive material 24 for use in this embodiment of the present invention, they may be formed from any conductive material suitable for supporting the drug delivery technique employed by the medical device, including iontophoresis and/or electroporation. Examples of conductive materials include suitable members of the following, among many others: metals and metal alloys (e.g., stainless steel or gold or platinum, due to their high conductivity, oxidation resistance, and radioopacity, which facilitates visibility of the device during fluoroscopy or the like, or magnesium, which can be left in the tissue where it will eventually oxidize *in vivo*), conductive polymers, semiconductors (e.g., silicon) including doped semiconductors, and conductive carbon. The conducting layer 24 may take on innumerable shapes, in addition to the preferred layer, including rods, wires, tubes, blades, and grids, among many others. The conducting layer 24 is configured such that, when the medical device is properly deployed in a subject and a suitable constant or variable voltage is applied between the conducting layer 24 and the body fluid 50, an electric field is generated such that the body fluid 50 is driven down into the channels 40 to contact the drug-containing layer 28. In certain embodiments, the therapeutic agent 29 is also electrically charged so that it is driven outward from the device and into the body fluid 50 under the influence of the electric field.

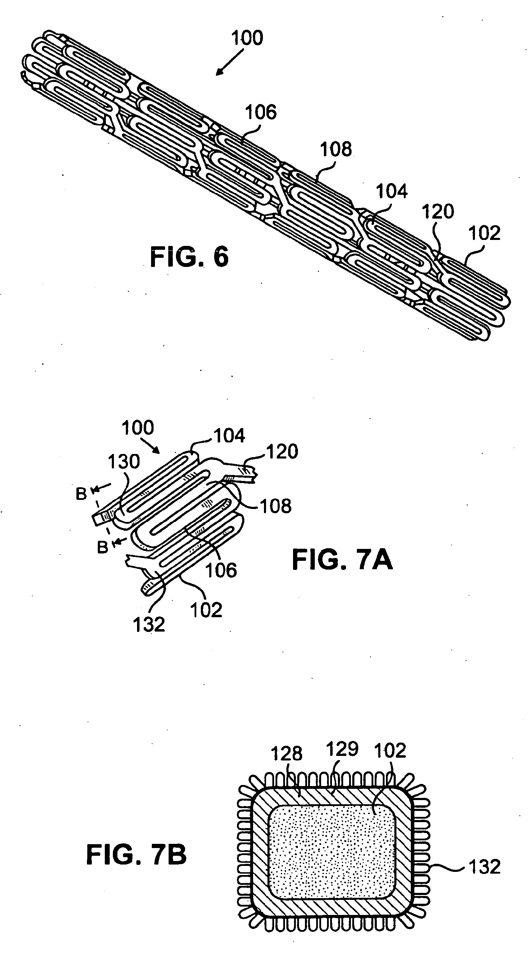

FIGS. 6 and 7 depict the incorporation of the present invention into a stent design. Now referring to FIG. 6, a stent 100 is shown in a perspective view, in a non-expanded form, in accordance with one embodiment of the present invention. The skeletal frame of the stent 100 preferably includes a structural network 102 that includes distinct, repetitive serpentine elements 108. Each serpentine element 108 consists of multiple U-shaped curves 104 and has no recognizable beginning or end. These U-shaped curves 104 form interstitial spaces 106. Due to its serpentine nature, each serpentine element 108 is radially expandable. Serpentine elements 108 are arranged along the longitudinal axis of the stent 100 so that the U-shaped curves 104 of abutting serpentine elements 108 may be joined via interconnecting elements 120, forming the stent 100.

As would be appreciated by one of ordinary skill in the art, the structural frame of the stent 100 can be of a variety of configurations and be made of any number of stent materials including metals, metal alloys, and polymeric materials, that perform the desired applications of radially expanding and maintaining the patency of various lumen passages within the human body, and all such variations are within the scope of the present invention, such as those manufactured by Boston Scientific Co[phi]oration, Natick, Massachusetts.

Referring now to FIG. 7A and FIG. 7B, a portion of the stent in FIG. 6 is depicted (in magnified cross-sectional view in FIG. 7B) with the surface-modulating region 132 disposed over at least a portion of the exterior surface of structural members 102. As described above for the catheter embodiments of the present invention, this surface-modulating region 132 is selectively applied to at least a portion of the exterior surface of the structural network 102 and may comprise, for example, a plurality of conductive columns coated with a hydrophobic dielectric layer. The stent 100, in certain embodiments, also includes a drug-containing layer 128 which includes a drug or therapeutic substance 129. Where the structural network 102 is formed of a conductive material, a separate conductor may not be required. The drug-containing layer 128 is associated with the surface-modulating region 132 as described above. As described above, once the stent is deployed within the body, an electrical potential may be applied, for example, between the structural network 102 and the surrounding bodily fluid (not shown). As a result, an electric field is generated, which results in contact between the drug-containing layer 128 and body fluid (not shown).

In those embodiments wherein the structural network 102 of the stent 100 is not made of a conductive material, such as a metal or a metal alloy, the structure of the stent 100 may include a conductive layer (not shown), which is capable of producing an electrical potential between the conductive layer and the surrounding bodily fluid, generating an electrical field through the surface-modulating region.

In certain preferred embodiments, the therapeutic substance is comprised, at least in part, of an anti-restenotic, anti -proliferative, and/or anti-angiogenic drug. For example, such a therapeutic agent can be dispersed and contained in a polymeric carrier matrix. As noted above, release of the therapeutic substance may be enhanced by contact with bodily fluids that are present in the blood or other body lumen. In some embodiments, the present invention is directed to implantable or insertable devices comprising neurostimulators, electrostimulators, and implantable electrodes. The present invention is also directed to implantable or insertable medical devices, such as the catheters and stents described above, but which do not have a drug-containing layer. For example, resistance to movement between a medical device and an adjacent solid may be reduced in either wet or dry conditions by providing the medical device with a low energy surface. Low energy surfaces are defined herein as surfaces that have hydrophobic static water contact angles (i.e., contact angles greater than 90[deg.], preferably 110° to 120° to 130° to 140° to 150° to 160° to 170° to 180°). FIGS. 8A and 8B show the behavior of a prior art low energy surface made of a layer of molecules on a positively charged and negatively charged substrate, respectively. The molecules have a hydrophobic chain portion and a charged polar portion.

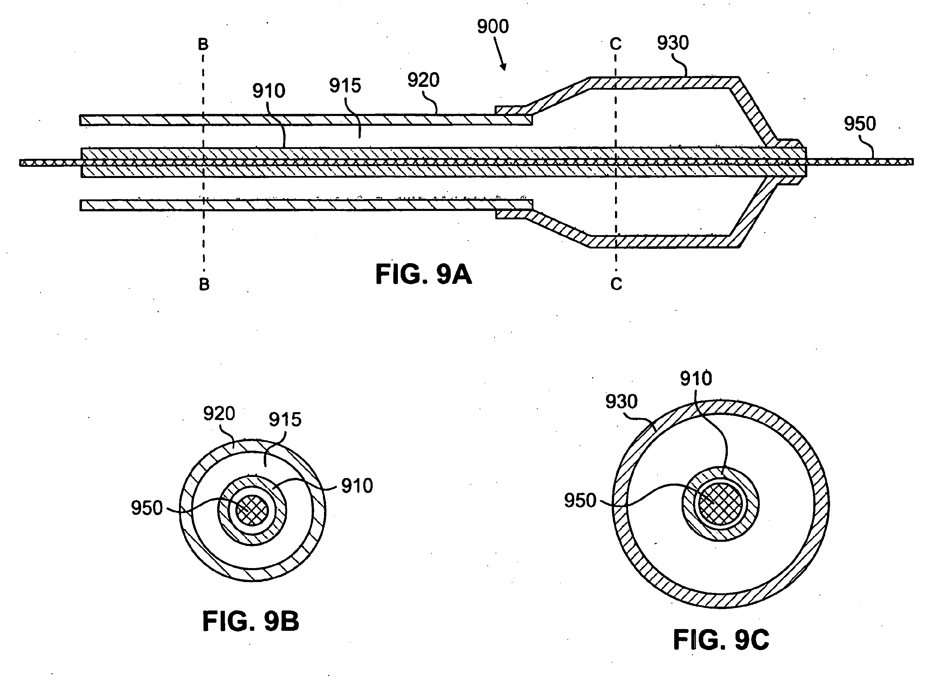

Referring now to Figs. 9A-9C in which the distal end of a guidewire-balloon catheter system is illustrated, this system includes a guidewire 950, which passes through a lumen formed by an inner tubular member 910. Also shown is an outer tubular member 920, which, along with inner tubular member 910, forms an annular inflation lumen 915 that provides for the flow of inflation fluid into balloon 930. Figs. 9B and 9C are schematic, axial, cross-sectional views of the balloon catheter of Fig. 9A, taken along planes B-B and C-C, respectively.

In such a system, it may be desirable to vary the surface friction at various times, including the friction that exists (a) between the inside surface of the member that forms the guidewire lumen of the catheter (e.g., inner tubular member 910) and the outside surface of the guidewire 950 over which it is passed, (b) between the outside surface of the balloon 930 and the vasculature, and/or (c) between the outside surface of the outer tubular member 920 and the vasculature. For this purpose, such surfaces may be rendered electrically actuatable between a hydrophobic state and a hydrophilic state in accordance with the present invention, for example, by providing a surface-modifying region such as those described elsewhere herein.

For example, to reduce friction during balloon 930 advancement and withdrawal along the guidewire 950, it may be desirable to provide the outside surface of the guidewire 950, the inside surface inner tubular member 910, the outside surface of the balloon 930, and the outside surface of the outer tubular member 920 with a hydrophobic surface, and preferably a superhydrophobic surface. During balloon inflation, on the other hand, it may be desirable to render one or more of these surfaces (e.g., the surface of the balloon 930, etc.) moderately hydrophilic (and preferably not so hydrophilic so as to create a slippery surface, for example, having a contact angle of 90° to 80° to 70° to 60°) so as to increase axial movement of the device within the body. This may be done by electrowetting the hydrophobic surface(s), as described elsewhere herein.

Similarly where a stent is deployed by a catheter such as balloon catheter 900, it may be desirable to electrowet the inside surface of the stent and/or the outside surface of the balloon by application of an electrical potential during stent advancement and expansion and, after stent expansion, to remove the potential and return the inside surface of the stent and/or the outside surface of the balloon to a hydrophobic or superhydrophobic state, allowing the balloon to be more readily withdrawn.

Although the above description has been applied to stents and catheters, it will be understood by one of ordinary skill in the art that the invention may be applicable to other insertable or implantable devices for use within the body, particularly those that come into contact with blood or body fluids.

For example, medical devices for use in conjunction with the present invention include a wide variety of implantable or insertable medical devices, which are implanted or inserted either for procedural uses or as implants. Examples include balloons, catheters (e.g., renal or vascular catheters such as balloon catheters), guide wires, filters (e.g., vena cava filters), stents (including coronary artery stents, peripheral vascular stents such as cerebral stents, urethral stents, ureteral stents, biliary stents, tracheal stents, gastrointestinal stents and esophageal stents), stent grafts, vascular grafts, vascular access ports, embolization devices including cerebral aneurysm filler coils (including Guglilmi detachable coils and metal coils), myocardial plugs, pacemaker leads, left ventricular assist hearts and pumps, total artificial hearts, heart valves, vascular valves, tissue bulking devices, anastomosis clips and rings, tissue staples and ligating clips at surgical sites, cannulae, metal wire ligatures, orthopedic prosthesis, joint prostheses, as well as various other medical devices that are adapted for implantation or insertion into the body.

The medical devices of the present invention include implantable and insertable medical devices that are used for diagnosis, for systemic treatment, or for the localized treatment of any tissue or organ. Non-limiting examples are tumors; organs including the heart, coronary and peripheral vascular system (referred to overall as "the vasculature"), the urogenital system, including kidneys, bladder, urethra, ureters, prostate, vagina, uterus and ovaries, eyes, lungs, trachea, esophagus, intestines, stomach, brain, liver and pancreas, skeletal muscle, smooth muscle, breast, dermal tissue, cartilage, tooth and bone. As used herein, "treatment" refers to the prevention of a disease or condition, the reduction or elimination of symptoms associated with a disease or condition, or the substantial or complete elimination of a disease or condition. Typical subjects (also referred to as "patients") are vertebrate subjects, more typically mammalian subjects and even more typically human subjects.

Although various embodiments are specifically illustrated and described herein, it will be appreciated that modifications and variations of the invention are covered by the above teachings and are within the purview of the appended claims. For example, radiofrequency transmitters for the medical devices are depicted herein, yet other means for energizing the device to modulate the electrowetting and other properties of the device are possible without departing from the scope of the present invention. Substitute means for energizing the device and which function similarly to radiofrequency transmitters will be obvious to one of ordinary skill in the art. Further, the above examples should not be interpreted to limit the modifications and variations of the invention covered by the claims but are merely illustrative of possible variations.

## Claims

1. A medical device comprising a surface region (30) that is electrically actuatable between a hydrophobic state and a less hydrophobic state or a hydrophilic state to bring a body fluid (50) into contact with a drug-containing region, said medical device being an implantable or insertable medical device, wherein said surface region (30) comprises conductive protrusions selected from conductive micro-protrusions, conductive nano-protrusions, or both, and a hydrophobic layer over said protrusions, wherein said drug-containing region (28) is disposed beneath said conductive protrusions.

2. The device of claim 1, wherein said surface region (30) comprises (A) a conductive region and (B) a layer of molecules that comprise (1) a hydrophobic chain portion that is covalently or non-covalently attached to said conductive region and (2) a charged polar portion, said charged polar portion (a) being drawn to the conductive region when the conductive region has a charge whose sign is opposite to that of the charged polar portion and (b) being repelled from the conductive region when the conductive region has a charge whose sign the same as that of the charged polar portion.

3. The device of claim 1, wherein said surface region (30) comprises: a conductive region and a dielectric layer disposed over said conductive region.

4. The device of claim 1, wherein said surface region (30) comprises said protrusions; and a hydrophobic dielectric layer over said protrusions.

5. The device of claim 1, wherein said surface region (30) comprises said protrusions; a dielectric layer over said protrusions; and a hydrophobic layer over said dielectric layer.

6. The device of claim 1, further comprising a drug-containing region between said conductive protrusions.

7. The device of claim 1, wherein the conductive protrusions comprise a semiconductor, a metal or a metal alloy.

8. The device of claim 1, wherein the hydrophobic layer comprises a fluorinated polymer or a silicone polymer or polytetrafluoroethylene or poly(dimethylsiloxane) or an elastomer.

9. The device of claim 5, wherein the conductive protrusions comprise silicon and the dielectric layer comprises silicon dioxide.

10. The device of claim 1, wherein the protrusions arc columns (32) have a diameter of 500 nm or less and a height of 10 µm or less.

11. The device of claim 10, wherein the distance between adjacent columns (32) is 5 µm or less.

12. The device of claim 1, wherein the medical device comprises an inflatable balloon-forming structure and the electrically actuatable surface region (30) is provided over at least a portion of a surface of the balloon-forming structure.

13. The device of claim 1, wherein the medical device comprises an expandable stent member and the electrically actuatable surface region (30) is provided over at least a portion of a surface of the stent member.

14. The device of claim 13, wherein the stent member comprises a material selected from a metal, a metal alloy, a polymer, or a combination thereof.

15. A medical device system comprising the implantable or insertable medical device of claim 1 or claim 2 or claim 3, an electrode configured for electrical contact with the body, and a power source in electrical communication with said conductive region and said electrode.

16. The medical device system of claim 15, wherein said electrode is provided on a surface of said medical device.

17. The medical device system of claim 15, wherein said electrode is implantable or insertable independent of said medical device.

18. The medical device system of claim 15, wherein said surface region (30) is superhydrophobic in the absence of an applied potential.

19. A method of modulating the lubricity of the medical device of claim 15 comprising applying an electrical potential from said power source that is sufficient to convert said surface region (30) from a hydrophobic state to a hydrophilic state.

20. The device of claim 15, wherein said drug-containing layer (28) comprises said drug (29) and a carrier matrix.

21. The device of claim 20, wherein the drug (29) contained in the drug-containing layer (28) is released into body fluid (50) upon application of a potential from said power source that is suitable to bring the body fluid (50) into contact with said drug-containing layer (28).

22. The device of claim 1, wherein said medical device is selected from catheters, stents, neurostimulators, electrostimulators and implantable electrodes.

23. The device of claim 1, wherein said medical device comprises a plurality of said surface regions (30).

## Patentansprüche

1. Medizinische Vorrichtung, umfassend einen Oberflächenbereich (30), der elektrisch zwischen einem hydrophoben Zustand und einem weniger hydrophoben Zustand oder einem hydrophilen Zustand antreibbar ist, um ein Körperfluid (50) in Kontakt mit einem Arzneimittel enthaltenden Bereich zu bringen, wobei die medizinische Vorrichtung eine implantierbare oder einfügbare medizinische Vorrichtung ist, wobei der Oberflächenbereich (30) leitende Vorwölbungen umfasst, die aus leitenden Mikro-Vorwölbungen, leitenden Nano-Vorwölbungen oder beidem ausgewählt sind, und eine hydrophobe Schicht über den Vorwölbungen, wobei der Arzneimittel enthaltende Bereich (28) unter den leitenden Vorwölbungen angeordnet ist.

2. Vorrichtung nach Anspruch 1, wobei der Oberflächenbereich (30) Folgendes umfasst: (A) einen leitenden Bereich und (B) eine Schicht Moleküle, die umfassen: (1) einen hydrophoben Kettenabschnitt, der kovalent oder nicht kovalent an dem leitenden Bereich befestigt ist, und (2) einen geladenen polaren Abschnitt, wobei der geladene polare Abschnitt (a) zum leitenden Bereich gezogen wird, wenn der leitende Bereich eine Ladung aufweist, deren Zeichen dem des geladenen polaren Abschnitts entgegengesetzt ist, und (b) von dem leitenden Bereich abgestoßen wird, wenn der leitende Bereich eine Ladung aufweist, deren Zeichen gleich dem des geladenen polaren Abschnitts ist.

3. Vorrichtung nach Anspruch 1, wobei der Oberflächenbereich (30) umfasst: einen leitenden Bereich und eine dielektrische Schicht, die über dem leitenden Bereich angeordnet ist.

4. Vorrichtung nach Anspruch 1, wobei der Oberflächenbereich (30) umfasst: die Vorwölbungen; und eine hydrophobe dielektrische Schicht über den Vorwölbungen.

5. Vorrichtung nach Anspruch 1, wobei der Oberflächenbereich (30) umfasst: die Vorwölbungen; eine dielektrische Schicht über den Vorwölbungen; und eine hydrophobe Schicht über der dielektrischen Schicht.

6. Vorrichtung nach Anspruch 1, überdies umfassend einen Arzneimittel enthaltenden Bereich zwischen den leitenden Vorwölbungen.

7. Vorrichtung nach Anspruch 1, wobei die leitenden Vorwölbungen umfassen: einen Halbleiter, ein Metall oder eine Metalllegierung.

8. Vorrichtung nach Anspruch 1, wobei die hydrophobe Schicht ein Fluorelastomer oder ein Silikonpolymer oder Polytetrafluorethylen oder Poly(dimethylsiloxan) oder ein Elastomer umfassen.

9. Vorrichtung nach Anspruch 5, wobei die leitenden Vorwölbungen Silizium umfassen und die dielektrische Schicht Siliziumdioxid umfasst.

10. Vorrichtung nach Anspruch 1, wobei die Vorwölbungs-Bogensäulen (32) einen Durchmesser von 500 nm oder weniger und eine Höhe von 10 µm oder weniger aufweisen.

11. Vorrichtung nach Anspruch 10, wobei der Abstand zwischen benachbarten Säulen (32) 5 µm oder weniger beträgt.

12. Vorrichtung nach Anspruch 1, wobei die medizinische Vorrichtung eine inflatierbare ballonbildende Struktur umfasst, und der elektrisch antreibbare Oberflächenbereich (30) über mindestens einen Abschnitt einer Oberfläche der ballonbildenden Struktur bereitgestellt ist.

13. Vorrichtung nach Anspruch 1, wobei die medizinische Vorrichtung ein expandierbares Stent-Element umfasst und der elektrisch antreibbare Oberflächenbereich (30) über mindestens einen Abschnitt einer Oberfläche des Stent-Elements bereitgestellt ist.

14. Vorrichtung nach Anspruch 13, wobei das Stent-Element ein Material umfasst, das aus einem Metall, einer Metalllegierung, einem Polymer oder einer Kombination daraus ausgewählt ist.

15. Medizinisches Vorrichtungssystem, umfassend die implantierbare oder einfügbare medizinische Vorrichtung nach Anspruch 1 oder Anspruch 2 oder Anspruch 3, eine Elektrode, die für den elektrischen Kontakt mit dem Körper ausgestaltet ist, und eine Stromquelle, die in elektrischer Verbindung mit dem leitenden Bereich und der Elektrode steht.

16. Medizinisches Vorrichtungssystem nach Anspruch 15, wobei die Elektrode auf einer Oberfläche der medizinischen Vorrichtung bereitgestellt ist.

17. Medizinisches Vorrichtungssystem nach Anspruch 15, wobei die Elektrode unabhängig von der medizinischen Vorrichtung implantierbar oder einfügbar ist.

18. Medizinisches Vorrichtungssystem nach Anspruch 15, wobei der Oberflächenbereich (30) in Abwesenheit eines angelegten Potenzials superhydrophob ist.

19. Verfahren des Modulierens der Gleitfähigkeit der medizinischen Vorrichtung nach Anspruch 15, umfassend das Anlegen eines elektrischen Potenzials von der Stromquelle, das ausreichend ist, um den Oberflächenbereich (30) von einem hydrophoben Zustand zu einem hydrophilen Zustand zu wandeln.

20. Vorrichtung nach Anspruch 15, wobei die Arzneimittel enthaltende Schicht (28) das Arzneimittel (29) und eine Trägermatrix umfasst.

21. Vorrichtung nach Anspruch 20, wobei das Arzneimittel (29), das in der Arzneimittel enthaltenden Schicht (28) enthalten ist, bei Anlegen eines Potenzials von der Stromquelle, das geeignet ist, das Körperfluid (50) in Kontakt mit der Arzneimittel enthaltenden Schicht (28) zu bringen, in das Körperfluid (50) freigegeben wird.

22. Vorrichtung nach Anspruch 1, wobei die medizinische Vorrichtung aus Kathetern, Stents, Neurostimulatoren, Elektrostimulatoren und implantierbaren Elektroden ausgewählt ist.

23. Vorrichtung nach Anspruch 1, wobei die medizinische Vorrichtung mehrere der Oberflächenbereiche (30) umfasst.

## Revendications

1. Dispositif médical comprenant une région de surface (30) qui est actionnable électriquement entre un état hydrophobe et un état moins hydrophobe ou un état hydrophile pour amener un fluide corporel (50) en contact avec une région contenant un médicament, ledit dispositif médical étant un dispositif médical implantable ou insérable, dans lequel ladite région de surface (30) comprend des saillies conductrices sélectionnées parmi des micro-saillies conductrices, des nano-saillies conductrices ou les deux, et une couche hydrophobe au-dessus desdites saillies, ladite région contenant un médicament (28) étant disposée au-dessous desdites saillies conductrices.

2. Dispositif selon la revendication 1, dans lequel ladite région de surface (30) comprend (A) une région conductrice et (B) une couche de molécules qui comprennent (1) une portion de chaîne hydrophobe qui est attachée de façon covalente ou non covalente à ladite région conductrice et (2) une portion polaire chargée, ladite portion polaire chargée (a) étant tirée vers la région conductrice quand la région conductrice a une charge dont le signe est opposé à celui de la portion polaire chargée et (b) étant repoussée de la région conductrice quand la région conductrice a une charge dont le signe est le même que celui de la portion polaire chargée.

3. Dispositif selon la revendication 1, dans lequel ladite région de surface (30) comprend : une région conductrice et une couche diélectrique disposée au-dessus de ladite région conductrice.

4. Dispositif selon la revendication 1, dans lequel ladite région de surface (30) comprend lesdites saillies ; et une couche diélectrique hydrophobe au-dessus desdites saillies.

5. Dispositif selon la revendication 1, dans lequel ladite région de surface (30) comprend lesdites saillies ; une couche diélectrique au-dessus desdites saillies ; et une couche hydrophobe au-dessus de ladite couche diélectrique.

6. Dispositif selon la revendication 1, comprenant également une région contenant un médicament entre lesdites saillies conductrices.

7. Dispositif selon la revendication 1, dans lequel les saillies conductrices comprennent un semi-conducteur, un métal ou un alliage métallique.

8. Dispositif selon la revendication 1, dans lequel la couche hydrophobe comprend un polymère fluoré ou un polymère de silicone ou du polytétrafluoroéthylène ou du poly(diméthylsiloxane) ou un élastomère.

9. Dispositif selon la revendication 5, dans lequel lesdites saillies conductrices comprennent du silicone et la couche diélectrique comprend du dioxyde de silicone.

10. Dispositif selon la revendication 1, dans lequel les colonnes d'arc des saillies (32) ont un diamètre de 500 nm ou moins et une hauteur de 10 µm ou moins.

11. Dispositif selon la revendication 10, dans lequel la distance entre des colonnes (32) adjacentes est égale ou inférieure à 5 µm.

12. Dispositif selon la revendication 1, dans lequel le dispositif médical comprend une structure formant un ballonnet gonflable, et la région de surface (30) actionnable électriquement est prévue au-dessus d'au moins une portion d'une surface de la structure formant un ballonnet.

13. Dispositif selon la revendication 1, dans lequel le dispositif médical comprend un élément de stent dilatable, et la région de surface (30) actionnable électriquement est prévue au-dessus d'au moins une portion d'une surface de l'élément de stent.

14. Dispositif selon la revendication 13, dans lequel l'élément de stent comprend un matériau sélectionné parmi un métal, un alliage métallique, un polymère ou une combinaison de ceux-ci.

15. Système de dispositif médical comprenant le dispositif médical implantable ou insérable selon la revendication 1 ou la revendication 2 ou la revendication 3, une électrode configurée pour un contact électrique avec le corps, et une source d'énergie en communication électrique avec ladite région conductrice et ladite électrode.

16. Système de dispositif médical selon la revendication 15, dans lequel ladite électrode est prévue sur une surface dudit dispositif médical.

17. Système de dispositif médical selon la revendication 15, dans lequel ladite électrode est implantable ou insérable indépendamment dudit dispositif médical.

18. Système de dispositif médical selon la revendication 15, dans lequel ladite région de surface (30) est super hydrophobe en l'absence d'application d'un potentiel.

19. Procédé de modulation du pouvoir lubrifiant du dispositif médical selon la revendication 15, comprenant l'application d'un potentiel électrique en provenance de ladite source d'énergie qui est suffisant pour convertir ladite région de surface (30) en la faisant passer d'un état hydrophobe à un état hydrophile.

20. Dispositif selon la revendication 15, dans lequel ladite couche contenant un médicament (28) comprend ledit médicament (29) et une matrice support.

21. Dispositif selon la revendication 20, dans lequel le médicament (29) contenu dans la couche contenant un médicament (28) est libéré dans le fluide corporel (50) lors de l'application d'un potentiel en provenance de ladite source d'énergie qui est approprié pour amener le fluide corporel (50) en contact avec ladite couche contenant un médicament (28).

22. Dispositif selon la revendication 1, dans lequel ledit dispositif médical est sélectionné parmi des cathéters, des stents, des neurostimulateurs, des électrostimulateurs et des électrodes implantables.

23. Dispositif selon la revendication 1, dans lequel ledit dispositif médical comprend une pluralité desdites régions de surface (30).
